# EUROPEAN PATENT APPLICATION

(11) **EP 2 210 534 A1**
(43) Date of publication of application: **28.07.2010**
(21) Application number: 09380195.9
(22) Date of filing: 22.12.2009
(51) Int. Cl.: A47G 9/10, A61F 5/058

(54) **Pillow for the prevention of cranial deformities in newborn**

(30) Priority: 22.01.2009 ES 200900122 U
(71) Applicant: Think Pipe Line S.L.N.E., 08240 Manresa, Barcelona (ES)
(72) Inventor: Verde Sanchez, David, 08240 Manresa, Barcelona (ES); Chern Law, Yin, 08240 Manresa, Barcelona (ES)
(74) Representative: Espiell Volart, Eduardo Maria

(57) **Abstract**

The pillow has an orifice of suitable dimensions, which decreases the local pressure exerted on the skull, the pillow is formed by a body of foam or another material deformable under pressure and a fabric coating which protects the core and improves the hold of the head, whose coating has an upper layer and another lower one sewn together in the same orifice and following its perimeter and at mid height of the foam core. The cavity formed has dimensions which adapt to the natural shape of the newborn baby's head.

## Description

### OBJECT OF THE INVENTION

The present invention relates to an anatomical pillow, whose clear purpose is to reduce the pressure exerted on the cranium of a newborn when it is lying down in the supine position, i.e., face up.

The specially designed shape of this pillow prevents all types of postural cranial deformations and helps to correct congenital deformations. The 3 main deformations are called Plagiocephaly, Brachycephaly and Scaphocephaly, depending on the shape adapted by the cranium.

Newborns spend a large part of the day lying down in supine position, whether it be sleeping, playing or travelling. The weight of the head itself causes a moulding of the bones against the contact surface, creating flat areas on the cranium. This pillow has been designed to support the head, minimizing the local pressures thereon and permitting a correct development of the baby's cranium.

### BACKGROUND OF THE INVENTION

In Asia, cushions stuffed with rice or soy seeds have been used for thousands of years to decrease the pressure on babies' craniums.

In western countries, newborns have traditionally slept in prone position, i.e. lying face down, so there is no precedent of this type. Nevertheless, since 1992 the American Academy of Pediatrics has recommended that babies less than one year-old be placed in supine position to reduce the incidence of Sudden Infant Death Syndrome.

The conventional pillows used to support the head of newborns feature a series of drawbacks amongst which must be pointed out the danger of suffocation due to the sponginess of the material, and the development of mites and other small insects which can cause allergic reactions in contact with the skin.

Also noteworthy is that the conventional pillows are manufactured with synthetic materials which may contain chemical products capable of producing allergic reactions when they make contact with the baby's skin.

### DESCRIPTION OF THE INVENTION

The pillow proposed has been devised to completely and satisfactorily resolve those drawbacks, being an anatomical pillow with suitable structural or configurative characteristics to, on the one hand, fulfil the function of supporting a lying down newborn on the pillow so that the pressure of the weight of the head itself is spread over a broad enough surface and so that the spinal column and the head are correctly aligned.

More specifically, the pillow of the invention exhibits the special feature of being formed by a flat foam body with a central orifice and a textile protection and support covering.

When the newborn supports their head in the orifice of the pillow, it is held at the foamy edges of the central orifice and also by the fabric which covers the foam. Therefore, a head support is achieved without high pressure points, which will prevent the appearance of flat areas on the cranium.

The centre of the pillow can be made up of several natural foamy materials such as, for example, latex, or artificial materials such as, for example, polyurethane foam. Likewise, more traditional stuffing can be used such as cotton or wool.

The central orifice can be complete, and even completely pass through the centre of the pillow, creating a ring-shaped pillow as shown in Figures 1 and 2. It can also be partial, creating a concave area where the head of the newborn can be rested, as shown in Figures 3 and 4.

The orifice or cavity (2 and 10) should preferably have a diameter of between 5 and 9 cm, and can exceed both limits to adapt to newborns of different ages and weights.

The part of the centre where the nape of the neck (3) rests should preferably have a width of between 4 and 6 cm, and can exceed both limits to adapt to newborns of different ages and weights.

The thickness of the centre of the pillow (4) shall preferably be between 3 and 5 cm and can exceed both limits to adapt to newborns of different ages and weights. The 2 faces (5, 6) of the centre can be parallel or offer a slight difference to offer greater comfort for the baby.

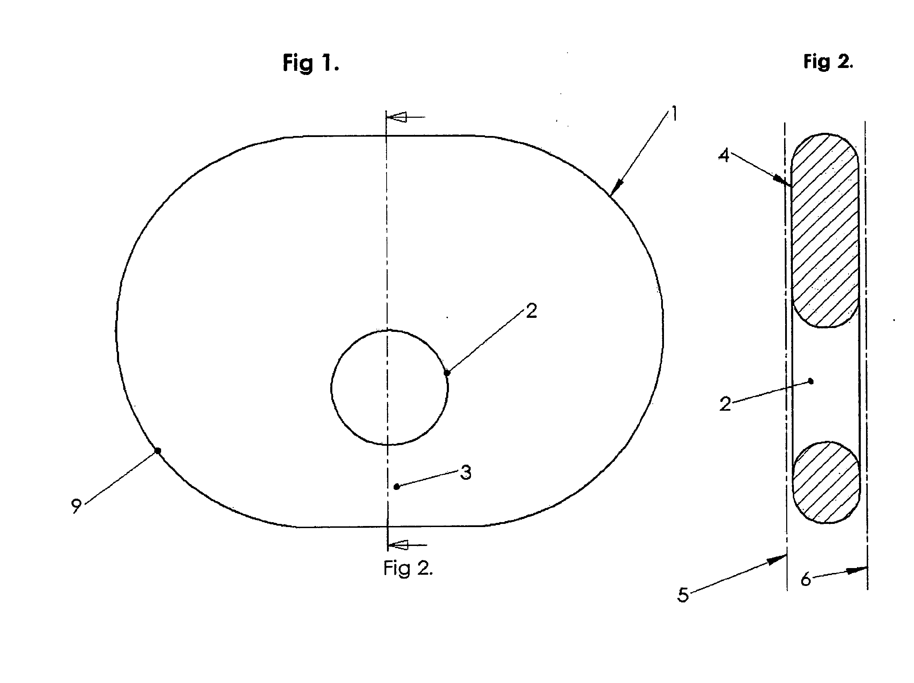

The centre of the pillow is covered with one or several layers of fabric (8) which fulfil the essential function of gently holding the head, sharing out the pressure in the area of the orifice and adapting to the specific shape of the user in the event that the orifice is whole (Figures 1 and 2). Both sides of this fabric are used to hold the head. The secondary function of this fabric is that of protecting the centre from possible vomiting and sweat brought about by the user.

In the version with the concave area, the lowest point thereof should preferably be between 7 and 10 cm from the outer edge of the pillow and this shall have a diameter of between 5 and 9 centimetres.

The outer perimeter of the pillow (9) can adopt any shape whether it be strictly decorative or specifically designed to adapt to a specific place such as, for example, a baby pram.

On the other hand, the pillow, in its version with orifice, exhibits the special feature of being reversible, where it can be used on both sides. This is an advantage during the warmer months when it can be more easily stained with sweat.

### DESCRIPTION OF THE DRAWINGS

In order to complement the description which is being carried out and with the purpose of helping towards a better understanding of the characteristics of the invention, in accordance with a preferred example of embodiment thereof, a set of drawings is attached as an integral part of said description, wherein the following, in an illustrative and non-limitative character, has been represented:
- fig. 1: shows a plan view of the centre of the pillow in its version with orifice in accordance with the object of the invention;
- fig. 2: shows a representation according to a profile view of what can be considered as a cross-section of Figure 1 made in accordance with the object of the invention;

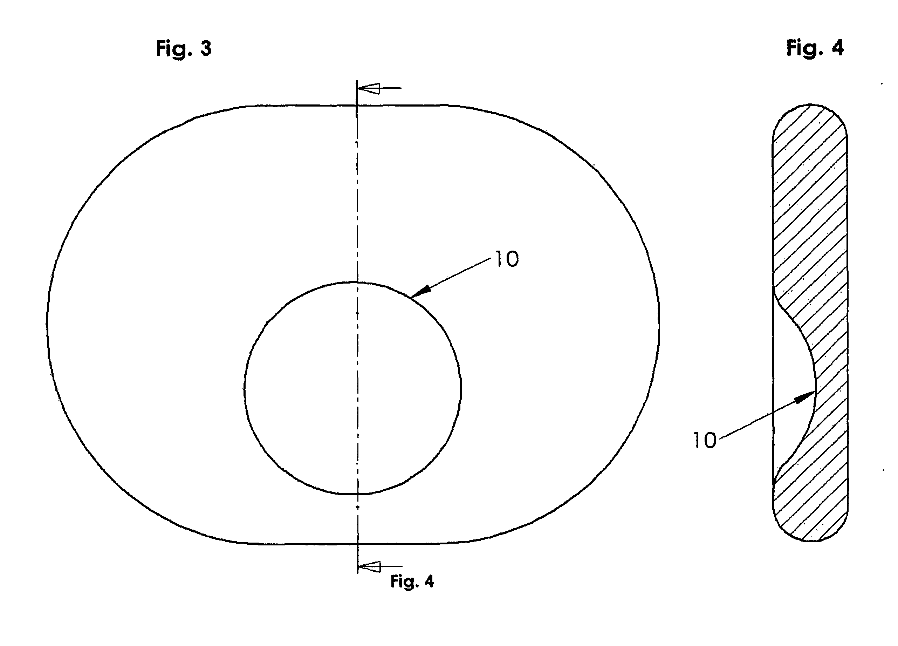

- fig. 3: shows a plan view of the pillow in its version with concave cavity in accordance with the object of the invention;
- fig. 4: shows a representation according to a profile view of what can be considered as a cross-section of Figure 3 made in accordance with the object of the invention;

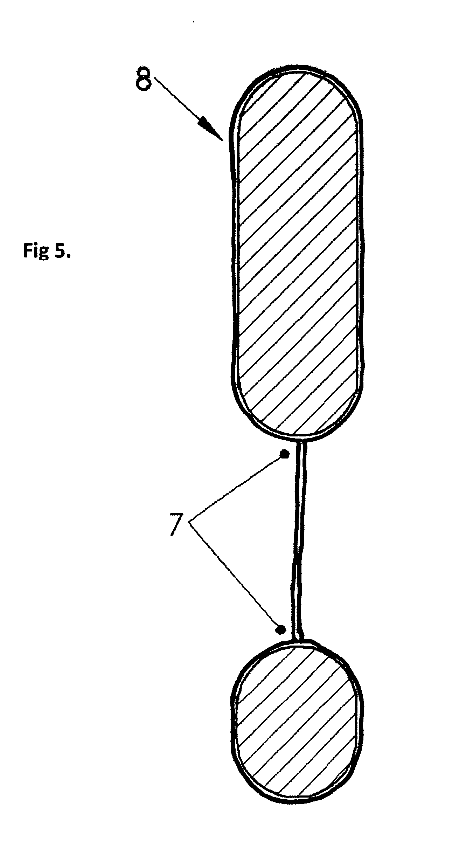

- fig. 5: shows a cross-section of the pillow with orifice and of the fabric (8) which covers it. The upper and lower fabrics are joined through the orifice and following the perimeter (7) thereof. This connection is preferably made at a medium height, leaving the fabric in the centre of the core;

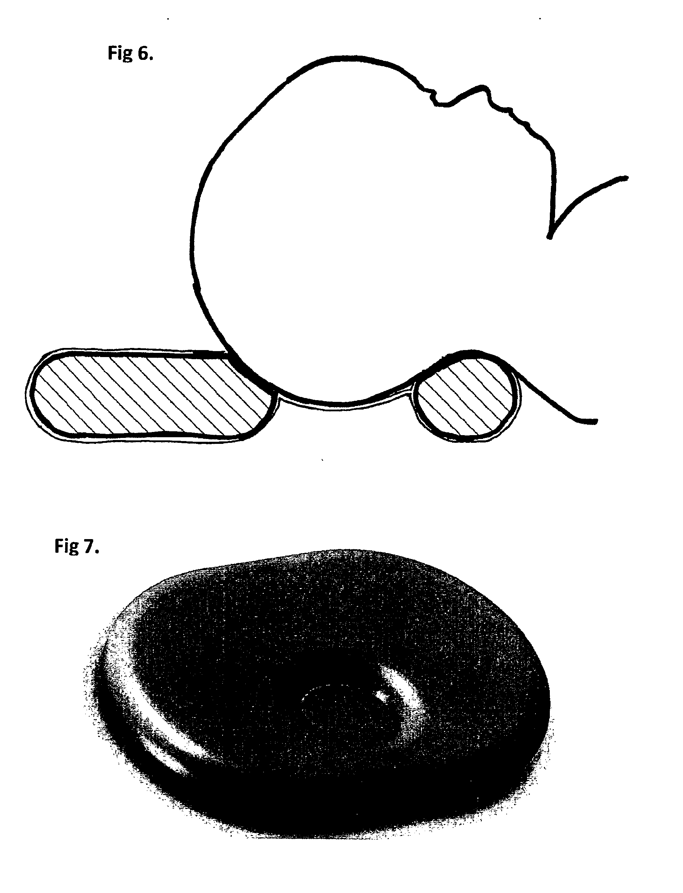

- Fig. 6: shows a newborn using the pillow and the shape taken by the foam and the fabric to adapt to the head;
- Fig.7: shows a schematic perspective image of the pillow object of the invention.

### PREFERRED EMBODIMENT OF THE INVENTION

In view of the figures, it can be observed how the anatomical pillow proposed is constituted from a single-piece body (1), of spongy material, solid, considerably level, of variable outer dimensions depending on the use of the pillow. The exterior shape is preferably an oval with diameters of 25 and 33 cm or a circle with an outer diameter of 22 cm.

Preferably, the pillow shall be made up by a 100% natural latex body and covered with a 100% cotton elastic fabric. In its main form, the pillow shall have an orifice (2) of between 6 and 8 cm in diameter. The width of the part of the ring intended to support the neck (3) shall preferably be 3 cm and with a thickness of 3 cm. The fabric shall be sewn achieving the perimeter of the orifice, thus joining the upper and lower layers.

On the other hand, the pillow will preferably exhibit the special feature that the foam centre is highly dense compared to traditional pillows. This can be situated between 70 and 130 Kg/m³.

It is not considered necessary to continue with this description for any person skilled in the art to understand the scope of the invention and the advantages derived therefrom.

The materials, shape, size and arrangement of the elements can be varied as long as this does not alter the essentiality of the invention.

The terms that have been used in this specification must always be taken in the broadest sense of the word and non-limitatively.

## Claims

1. ANATOMIC PILLOW, DESIGNED TO PERMIT A CORRECT POSITIONING AND SUPPORT OF THE HEAD OF A NEWBORN, by means of an orifice of previously described dimensions which decrease the local pressure exerted on the cranium, which is formed by means of a foam body or body of other material which can be deformed under pressure and a fabric cover which protects the centre and improves the holding of the head. To do this, the upper and lower layers of said fabric are sewn together in the orifice, following the perimeter thereof at a medium height of the foamy centre.

2. The Pillow of claim 1, wherein the fabric offers sufficient support due to its own physical, elastic or contractive properties, and wherein the lower and upper layers do not need to be joined along the perimeter of the orifice.

3. ANATOMIC PILLOW, DESIGNED TO PERMIT A CORRECT POSITIONING AND SUPPORT OF THE HEAD OF A NEWBORN, which provides a cavity of dimensions such that it adapts to the natural shape of the head of a newborn, with the aim of decreasing local pressure points on its head.

4. The Pillow of claim 3, which is **characterized in that** it is covered by a layer of fabric which protects the centre of the pillow.

5. The Pillow of claims 1 and 3 wherein the outer protective fabric is permanently attached to the pillow without the possibility of removal without destroying the pillow.

6. The Pillow of claims 1 and 3, which is **characterized in that** it has the upper and lower faces of the foamy centre arranged in parallel to each other or with a slight inclination to improve the comfort of the newborn.

7. The Pillow of claims 1 and 3, which is **characterized in that** it has a strictly decorative shape on its outer perimeter or one that is adapted for a specific use such as for a cradle or a baby pram.

8. The Pillow of claims 1 and 3, which is **characterized in that** it is manufactured from natural foamy latex, and by heat moulding according to the current state of the art.
